# EUROPÄISCHE PATENTSCHRIFT

(11) **EP 1 155 657 B1**
(45) Veröffentlichungstag und Bekanntmachung des Hinweises auf die Patenterteilung: **29.11.2006**
(21) Anmeldenummer: 00110768.9
(22) Anmeldetag: 19.05.2000
(51) Int. Cl.: A61B 5/00

(54) **Vorrichtung und Verfahren zur Erkennung von tumorösem Gewebe**
Apparatus and method for distinguishing cancerous tissue
Appareil et procédé pour détecter les tissus cancéreux

(43) Veröffentlichungstag der Anmeldung: 21.11.2001
(73) Patentinhaber: Coherent GmbH, 37079 Göttingen (DE)
(72) Erfinder: Hohla, Alexander, 80789 München (DE); Leipert, Gunther, 82205 Gilching (DE)
(74) Vertreter: Hofstetter, Alfons J.

(56) Entgegenhaltungen:
- EP-A- 0 512 965
- WO-A-90/10219
- GB-A- 2 203 831
- US-A- 4 449 535
- US-A- 4 786 813
- US-A- 5 131 398
- ZONIOS G I ET AL: "MORPHOLOGICAL MODEL OF HUMAN COLON TISSUE FLUORESCENCE" IEEE TRANSACTIONS ON BIOMEDICAL ENGINEERING,US,IEEE INC. NEW YORK, Bd. 43, Nr. 2, 1. Februar 1996 (1996-02-01), Seiten 113-122, XP000628420 ISSN: 0018-9294

## Beschreibung

Die vorliegende Erfindung bezieht sich auf eine Vorrichtung zur Erkennung von tumorösem Gewebe mit mindestens einer ersten Anregungslichtquelle, wobei die erste Anregungslichtquelle ein erstes Anregungslicht einer Wellenlänge zwischen 300 - 314 nm aussendet und mindestens einem Lichtleiter zur Führung des ersten Anregungslichts an ein Objektfeld des zu untersuchenden Gewebes aufweist und mit mindestens einem Objektiv zur Abbildung eines mittels des ersten Anregungslichts generierten Autofluoreszenzsignals und/oder Remissionssignals des Gewebes auf einen CCD-Chip oder ICCD-Chip einer Kamera sowie mindestens eine Datenverarbeitungsanlage zur Verarbeitung der von der Kamera übermittelten Signale. Die Erfindung betrifft zudem ein Verfahren zur Erkennung von tumorösem Gewebe.

Die Früherkennung von Tumoren ist die bedeutendste Voraussetzung zu ihrer wirksamen Bekämpfung. Die Heilungschancen von Tumorpatienten werden dadurch entscheidend verbessert. Bekannte Vorrichtungen und Verfahren zur Erkennung von tumorösem Gewebe beziehen sich auf zwei unterschiedliche Diagnosemethoden. Einerseits wird auf der Basis der laserinduzierten Fluoreszenz unter Verabreichung von synthetischen Fluoreszenzfarbstoffen oder synthetischen Porphyringemischen versucht entsprechende Tumore zu markieren und darzustellen. Diese Methode weist jedoch den Nachteil auf, daß durch die genannten synthetischen Substanzen eine Hautsensibilisierung erfolgt, so daß die Patienten über mehrere Wochen vor intensiver Lichteinstrahlung geschützt werden müssen. Zudem verhindert ein geringer Fluoreszenzquantenwirkungsgrad eine gesicherte Unterscheidung zwischen gesunden und kranken Gewebe. Es wurde daher versucht über die gewebeeigene UV-angeregte Autofluoreszenz Informationen zur Unterscheidung zwischen gesunden und tumorösem Gewebe zu erhalten. So wird in der US-A-5 131 398 und der WO 97 06724 A die spektroskopische Erkennung von Tumoren und Neoplasieen mit UV-Licht zwischen 300 und 312 nm ausführlich beschrieben. Dabei wird mittels UV-Licht in dem genannten Wellenlängenbereich die gewebeeigene Fluoreszenz erzeugt und über einen Spektrometer spektral aufgelöst und dargestellt. Das Gewebe wird dabei punktuell mit einer entsprechenden Lichtsonde abgetastet. Die US-A-5 131 398 offenbart als signifikantes Merkmal zur Unterscheidung zwischen normalem, gesundem Gewebe und tumorösem Gewebe das Intensitätsverhältnis der Autofluoreszenz in den Fluoreszenzmaxima bei 340 nm und 440 nm. Die Aufnahme und Weiterverarbeitung von Bildern wird nicht beschrieben, es wird lediglich ein punktuelles Abscannen und Untersuchen des bestrahlten Gewebes offenbart. Die EP-A-0 512 965 beschreibt ein endoskopisches Bildverarbeitungssystem bei dem mit einer Laserlichtquelle oder mit einer Xenonlichtquelle erzeugtes Licht in einem Lichtleiter an das zu untersuchende Gewebe geführt wird. Das vom Gewebe abgegebene Licht wird u.a. von einem Prisma in mindestens zwei Bilder aufgeteilt, die dann von einer entsprechenden Anzahl von CCD-Kameras aufgenommen werden. Hierzu befinden sich vor jeder Kamera entsprechende Filter. Es werden keine UV-Bilder aufgenommen bzw. verarbeitet. Gleiches gilt für das in einem Artikel von Zonios G. I. et al. ("Morphological model of human colon tissue fluorescence", In: IEEE Transactions on biomedical engineering, Vol. 43. No. 2, Feb. 1996, Seiten 112-113) veröffentlichte Verfahren bzw. die entsprechende Vorrichtung. Es erfolgt keine Aufspaltung des rückgestreuten oder emittierten Lichts in mehrere Bilder mit unterschiedlichen Wellenlängen oder -bereichen. Aus der US-A-4 786 813 und der WO 90 10219 A sind ebenfalls jeweils Bildaufnahmesysteme zur Darstellung von Fluoreszenzen beschrieben.

Die bekannten Vorrichtungen und Verfahren zur Darstellung von tumorösem Gewebe mit Hilfe der Autofluoreszenz des Gewebes weisen jedoch den Nachteil auf, daß man keinen visuellen Eindruck des gesamten Tumorareals bzw. des gesamten untersuchten Gewebeareals erhält.

Es ist daher Aufgabe der vorliegenden Erfindung, eine gattungsgemäße Vorrichtung und ein gattungsgemäßes Verfahren bereitzustellen, dass eine Visualisierung des gesamten untersuchten Gewebebereichs bei einer deutlichen Unterscheidung von normalem, gesundem Gewebe von tumorösem Gewebe gewährleistet.

Gelöst wird diese Aufgabe durch eine gattungsgemäße Vorrichtung mit den Merkmalen des Anspruches 1 sowie ein gattungsgemäßes Verfahren mit den Merkmalen des Anspruchs 5. Vorteilhafte Ausgestaltungen sind in den Unteransprüchen beschrieben.

Eine erfindungsgemäße Vorrichtung zur Erkennung von tumorösem Gewebe weist mindestens eine erste Anregungslichtquelle auf, wobei die erste Anregungslichtquelle ein erstes Anregungslicht einer Wellenlänge zwischen 300 - 314 nm aussendet und zudem mindestens einem Lichtleiter zur Führung des ersten Anregungslichts an ein Objektfeld des zu untersuchenden Gewebes ausgebildet ist die Vorrichtung mindestens ein Objektiv zur Abbildung eines mittels des ersten Anregungslichts generierten Autofluoreszenzsignals und/oder Remissionssignals des Gewebes auf einen CCD-Chip oder ICCD-Chip einer Kamera sowie mindestens eine Datenverarbeitungsanlage zur Verarbeitung der von der Kamera übermittelten Signale umfaßt, wobei das Objektiv ein Dachkantprisma sowie mindestens eine achromatische UV-Linse aufweist und geeignet ist UV-Licht zu verarbeiten und derart ausgestaltet ist, dass mindestens zwei Bilder aus unterschiedlichen Spektralbereichen des fluoreszierenden Objektfelds generiert und auf den CCD-Chip oder ICCD-Chip abgebildet werden. Dabei stellt mindestens jeweils ein Bild den UV-Bereich und einen anderen, davon verschiedenen Wellenlängen-Bereich des Autofluoreszenzsignals und/oder des Remissionssignals des Objektfelds dar. Damit ist gewährleistet, dass eine Visualisierung des gesamten untersuchten Gewebebereichs bei einer deutlichen Unterscheidung von normalem, gesundem Gewebe von tumorösem Gewebe erfolgt. Eine derartige Anordnung gewährleistet zudem die exakte Auftrennung und Darstellung des beleuchteten Objektfelds in mehrere Bilder unterschiedlicher Wellenlängen-Bereiche des Autofluoreszenzsignals und/oder des Remissionssignals des Objektfelds.

In einer weiteren vorteilhaften Ausgestaltung der Erfindung weist die Vorrichtung eine zweite Anregungslichtquelle zur Aussendung von einem zweiten Anregungslicht mit einer Wellenlänge zwischen 312 - 340 nm und/oder 350 - 410 nm auf. Durch die Zuschaltung einer zweiten Anregungslichtquelle mit einem zum ersten Anregungslicht unterschiedlichen zweiten Anregungslicht ist es möglich, daß weitere Bildinformationen des Objektfelds ermittelt und verarbeitet werden können. Dies erhöht in bestimmten Fällen die Selektivität der Vorrichtung bei der Unterscheidung von normalem Gewebe und tumorösem Gewebe.

Ein erfindungsgemäßes Verfahren zur Erkennung von tumorösem Gewebe weist folgende Verfahrensschritte auf: (a) Bestrahlung von Gewebe mit einem ersten Anregungslicht einer Wellenlänge zwischen 300 - 314 nm einer ersten Anregungslichtquelle und Generierung einer Autofluoreszenz und/oder Remission eines Objektfelds des bestrahlten Gewebes; (b) Generierung von mindestens zwei Bildern aus unterschiedlichen Spektralbereichen des fluoreszierenden Objektfelds mittels eines Objektivs einer Kamera, wobei das Objektiv ein Dachkantprisma sowie mindestens eine achromatische UV-Linse aufweist und Abbildung der mindestens zwei Bilder auf einen CCD-Chip oder ICCD-Chip der Kamera, wobei mindestens jeweils ein Bild den UV-Bereich und einen anderen, davon verschiedenen Wellenlängen-Bereich des Autofluoreszenzsignals und/oder des Remissionssignals des Objektfelds darstellen; (c) Übermittlung der in der Kamera generierten Bild-/Videosignale an eine Datenverarbeitungsanlage; (d) Subtraktion von Backgroundsignalen von den generierten Bild-/Videosignalen; (e) Einlesen und Verrechnen des UV-Bildes in und mit einem Blaukanal und Einlesen und Verrechnen des anderen Bildes in und mit einem Grün- und/oder Rotkanal; (f) Verstärkung oder Abschwächung der einzelnen Farbkanäle zur Erzielung einer Standard-Farbeinstellung für normales, nicht-tumoröses Gewebe; und (g) Auswertung der farbcodierten Bilder bzw. deren farbcodierten Bild-/Videosignalen zur Unterscheidung von normalem, gesundem Gewebe von tumorösem Gewebe. Damit ist wiederum gewährleistet, dass eine Visualisierung des gesamten untersuchten Gewebebereichs bei einer deutlichen Unterscheidung von normalem, gesundem Gewebe von tumorösem Gewebe erfolgt.

In einer vorteilhaften Ausgestaltung des erfindungsgemäßen Verfahrens werden im Verfahrensschritt (b) zwei Bilder generiert, wobei ein Bild den UV-Bereich und das andere Bild den sichtbaren Wellenlängen-Bereich des Autofluoreszenzsignals und/oder des Remissionssignals des Objektfelds darstellt. Durch die anschließend erfolgende Farbkodierung der beiden Bilder ergibt sich eine genaue Darstellung und Quantifizierung der unterschiedlichen Gewebearten. In einer weiteren vorteilhaften Ausgestaltung des erfindungsgemäßen Verfahrens wird im Verfahrensschritt (a) das Gewebe mit der ersten Anregungslichtquelle und einer zweiten Anregungslichtquelle zur Aussendung von einem zweiten Anregungslicht mit einer Wellenlänge zwischen 312 - 340 nm und/oder 350 - 410 nm bestrahlt. Durch die Bestrahlung mit einer zweiten Anregungslichtquelle mit einem zum ersten Anregungslicht unterschiedlichen zweiten Anregungslicht ist es möglich, daß weitere Bildinformationen des Objektfelds ermittelt und verarbeitet werden können. Dies erhöht in bestimmten Fällen die Selektivität der Vorrichtung bei der Unterscheidung von normalem Gewebe und tumorösem Gewebe. So können zum Beispiel im Verfahrensschritt (b) zwei Bilder generiert werden, wobei ein erstes Bild den UV-Bereich und ein anderes Bild den Infrarot-Bereich des Autofluoreszenzsignals und/oder des Remissionssignals des Objektfelds darstellt. Es wird dadurch aber auch möglich, daß im Verfahrensschritt (b) drei Bilder generiert werden, wobei ein erstes Bild den UV-Bereich, das zweite Bild den sichtbaren Wellenlängen-Bereich und das dritte Bild den Infrarot-Bereich des Autofluoreszenzsignals und/oder des Remissionssignals des Objektfelds darstellt. In einer weiteren vorteilhaften Ausgestaltung des erfindungsgemäßen Verfahrens erfolgt die Auswertung der farbcodierten Bilder bzw. farbcodierten Bild-/Videosignale im Verfahrensschritt (g) durch Vergleich und Darstellung der generierten Standard-Farbeinstellung für normales, nicht-tumoröses Gewebe mit den generierten Farbeinstellungen für tumoröses Gewebe. Dadurch ist eine Visualisierung des gesamten untersuchten Gewebebereichs bei einer deutlichen Unterscheidung zwischen normalem und tumorösem Gewebe gewährleistet.

In einer weiteren vorteilhaften Ausgestaltung des erfindungsgemäßen Verfahrens umfasst die Auswertung der farbcodierten Bilder bzw. farbcodierten Bild-/Videosignale im Verfahrensschritt (g) folgende Schritte: (g1) Aufheben der Gammakorrektur durch eine Korrekturfunktion und Linearisierung der Farbkanäle; (g2) Verhältnisbildung der Intensitäten der Farbkanäle und Logarithmierung des Ergebnisses, wobei eine Charakterisierung des Gewebes dahingehend erfolgt, daß positive Werte tumoröses Gewebe und negative Werte normales, gesundes Gewebe darstellen; und (g3) Mittelung der Helligkeitswerte der Farbkanäle und Verrechnung mit einem in Verfahrensschritt (g2) erstelltem Ratiobild zur Darstellung der Geometrie der Verteilung der Gewebearten. Diese Art der Darstellung und Auswertung ermöglicht eine sehr genaue Quantifizierung der unterschiedlichen Gewebearten.

Weitere Aufgaben, Vorteile und Merkmale der vorliegenden Erfindung ergeben sich aus den folgenden, in den Zeichnungen dargestellten Ausführungsbeispielen.
- Figur 1: ist eine schematische Darstellung der erfindungsgemäßen Vorrichtung;
- Figur 2: zeigt eine vereinfachte Darstellung des Strahlengangs in einem Teil der erfindungsgemäßen Vorrichtung;
- Figur 3: zeigt ein Ablaufdiagramm eines Ausführungsbeispiels des erfindungsgemäßen Verfahrens; und
- Figur 4: zeigt ein Ablaufdiagramm eines weiteren Ausführungsbeispiels des erfindungsgemäßen Verfahrens.

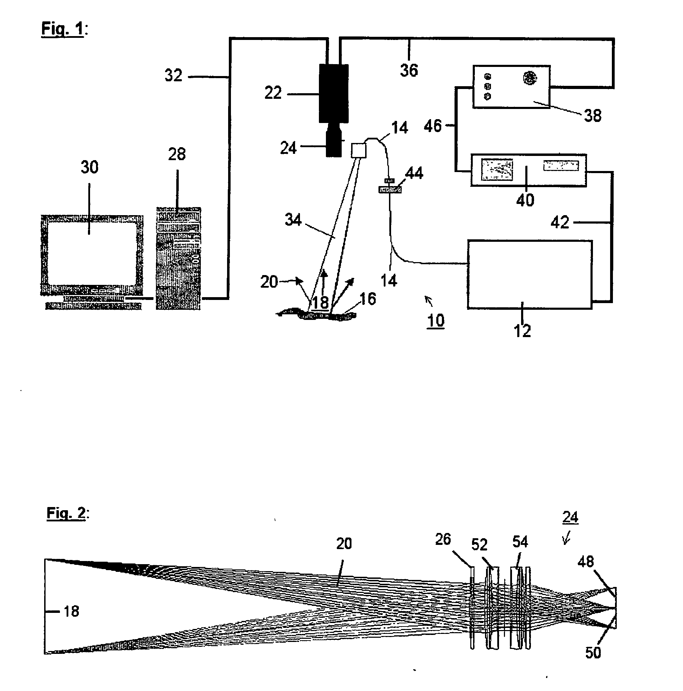

Die Figur 1 zeigt in einer schematischen Darstellung eine Vorrichtung 10 zur Erkennung von tumorösem Gewebe mit einer ersten Anregungslichtquelle 12, welche über einen Lichtleiter 14 ein erstes Anregungslicht 34 einer Wellenlänge zwischen 300 - 314 nm aussendet. Das erste Anregungslicht 34 wird dabei an ein Objektfeld 18 eines zu untersuchenden Gewebes 16 geführt. Ein durch das erste Anregungslicht 34 generiertes Autofluoreszenzsignal und/oder Remissionssignal 20 wird über ein Objektiv 24 auf einen CCD-Chip oder ICCD-Chip einer Kamera 22 abgebildet. Über eine Datenleitung 32 ist die Kamera 22 mit einer Datenverarbeitungsanlage 28 verbunden. In der Datenverarbeitungsanlage 28 werden die von der Kamera 22 generierten Signale verarbeitet und auf einem Bildschirm 30 dargestellt. Es ist aber auch möglich, andere Vorrichtungen zur bildgebenden Darstellung mit der Datenverarbeitungsanlage 28 zu verbinden.

Des weiteren erkennt man, daß dem Lichtleiter 14 ein Faserrüttler 44 zwischengeschaltet ist. Die erste Anregungslichtquelle 12 ist in dem Ausführungsbeispiel ein XeCl-Laser. Es ist aber auch möglich, daß die Anregungslichtquelle ein XeF-Laser oder ein HeCd-Laser ist. Mit dem XeCl-Laser wird das Gewebe 16 zur Autofluoreszenz angeregt. Zeitgleich mit dem Laserimpuls wird ein Sync-out-Signal erzeugt, welches über eine Signalleitung 42 zu einem Pulsgenerator 40 übertragen wird. Der Pulsgenerator 40 verzögert das Sync-out-Signal und überträgt das verzögerte Signal über eine Datenleitung 46 an eine Steuereinheit 38 der Kamera 22. Das verzögerte Sync-out-Signal wird dabei als Triggersignal über die Triggersignalleitung 36 an die Kamera 22 übermittelt. Das Bild-/Videosignal der Kamera 22 wird dann über die Datenleitung 32 an eine Videokarte der Datenverarbeitungsanlage 28 für die Bildverarbeitung weitergeleitet.

In einem nicht dargestellten weiteren Ausführungsbeispiel ist es möglich, daß die Vorrichtung 10 eine zweite Anregungslichtquelle zur Aussendung von einem zweiten Anregungslicht mit einer Wellenlänge zwischen 312 - 340 nm und/oder 350 - 410 nm aufweist. Als Anregungslichtquelle hierfür kann eine Xe-, Hg- oder Deuterium-Lampe oder ein N₂-Laser dienen.

Figur 2 zeigt eine vereinfachte Darstellung des Strahlengangs in einem Teil der Vorrichtung 10. Man erkennt, daß das Objektiv 24 der Kamera 22 ein Dachkantprisma 26 und ein achromatisches UV-Linsenpaar 52, 54 aufweist. Dadurch wird die vom Objektfeld 18 ausgehende Autofluoreszenz und/oder Remission 20 des Gewebes 16 in zwei Bilder 48,50 unterteilt. Die beiden Bilder 48,50 werden aus unterschiedlichen Spektralbereichen des fluoreszierenden Objektfelds 18 generiert. Das Bild 48 stellt dabei den UV-Bereich des Autofluoreszenzsignals und/oder Remissionssignals 20 des Objektfelds 18 dar. Das Bild 50 spiegelt in dem Ausführungsbeispiel den sichtbaren Bereich des Autofluoreszenzsignals und/oder Remissionssignals 20 wieder. Das Objektiv 24 ist dabei geeignet UV-Licht zu verarbeiten. Es ist aber auch möglich, daß die Aufteilung in zwei Bilder unter Verwendung anderer optisch abbildender Elemente erfolgt. Es ist auch denkbar, daß die Aufteilung rechnerisch direkt im CCD-Chip oder ICCD-Chip der Kamera erfolgt.

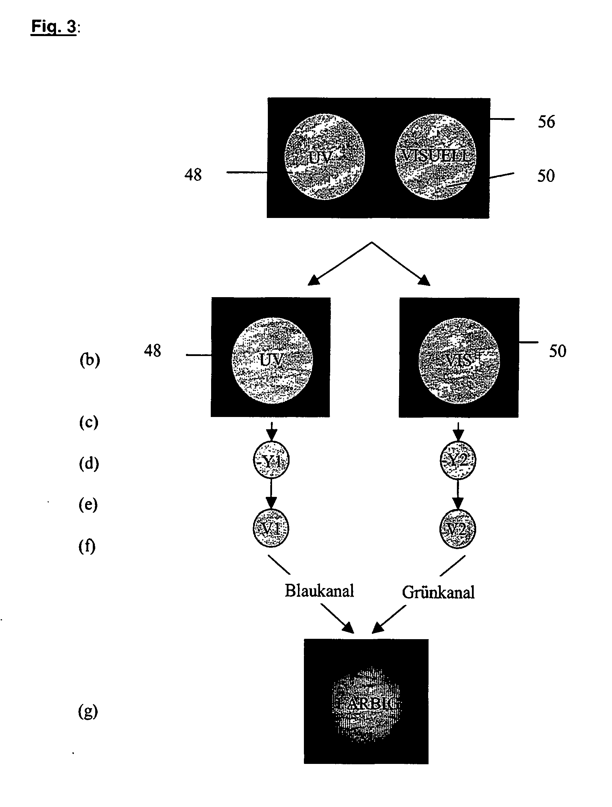

Figur 3 zeigt ein Ablaufdiagramm eines Ausführungsbeispiels des erfindungsgemäßen Verfahrens. Man erkennt, daß nach einem ersten Verfahrensschritt (a), nämlich der Bestrahlung des Gewebes 16 mit dem ersten Anregungslicht 34 und der Generierung der Autofluoreszenz und/oder Remission des Objektfelds 18 zwei Bilder 48, 50 als Schwarz/Weiß-Doppelbild 56 generiert werden. Das Bild 48 stellt dabei den UV-Bereich und das Bild 50 den sichtbaren Wellenlängen-Bereich des Autofluoreszenzsignals und/oder Remissionssignals 20 dar. Die Abbildung erfolgt dabei auf den CCD-Chip oder ICCD-Chip der Kamera 22. Die Signaldaten werden dann aus den Chips ausgelesen und an die Datenverarbeitungsanlage 28 übertragen. Dabei werden die Backgroundsignale Y1 und Y2 von den generierten Bild-/Videosignalen der Einzelbilder jeweils abgezogen. Dann erfolgt die Einlesung und Verrechnung der Bilder 48, 50 in den Blau- bzw. Grünkanal sowie eine Verstärkung oder Abschwächung der einzelnen Farbkanäle zur Erzielung einer Standardfarbeinstellung für normales, nicht-tumoröses Gewebe. Bei gleicher Intensität der beiden Farbkanäle würde nämlich die Mischfarbe Zyan im verrechneten Bild entstehen. Da aber auch für normales Gewebe unterschiedliche Intensitätsverhältnisse von UV- und sichtbarer Fluoreszenz und/oder Remission vorliegen, muß eine Verstärkung oder Abschwächung erfolgen, sodaß für normales Gewebe das genannte zyanfarbige Bild entsteht. Es werden Bilder V1 und V2 generiert. Ein vorliegendes Tumorgewebe demarkiert sich dann bei dieser Standardeinstellung als bläuliches Areal, da hier der UV-Anteil erhöht bzw. der sichtbare Anteil erniedrigt ist. Die Auswertung der farbcodierten Bilder bzw. farbcodierten Bild-/Videosignale erfolgt daher im Verfahrensschritt (g) durch Vergleich und Darstellung der generierten Standard-Farbeinstellung für normales Gewebe mit den generierten Farbeinstellungen für tumoröses Gewebe.

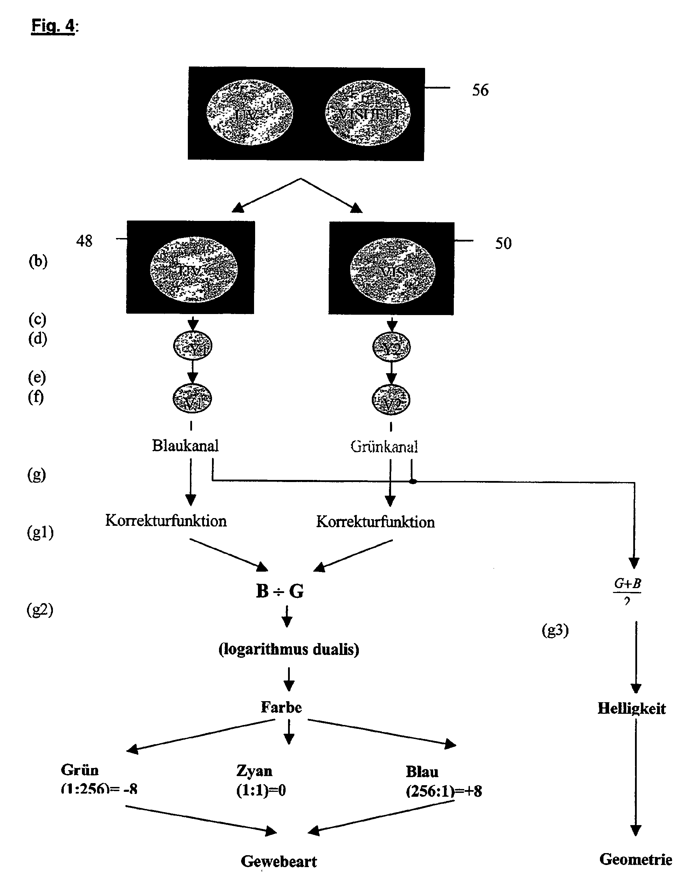

Figur 4 zeigt ein Ablaufdiagramm eines weiteren Ausführungsbeispiels des erfindungsgemäßen Verfahrens. Hierbei erfolgt die Auswertung der farbcodierten Bilder bzw. farbcodierten Bild-/Videosignale im Verfahrensschritt (g) mittels der Aufhebung der Gammakorrektur durch eine Korrekturfunktion und Linearisierung der Farbkanäle (Verfahrensschritt (g1)). Zudem erfolgt im Verfahrensschritt (g2) eine Verhältnisbildung der Intensitäten der Farbkanäle und Logarithmierung des Ergebnisses, wobei eine Charakterisierung des Gewebes dahingehend erfolgt, daß positive Werte tumoröses Gewebe und negative Werte normales, gesundes Gewebe darstellen. Parallel dazu erfolgt im Verfahrensschritt (g3) eine Mittelung der Helligkeitswerte der Farbkanäle und eine Verrechnung mit einem in Verfahrensschritt (g2) erstellten Ratiobild zur Darstellung der Geometrie der Verteilung der Gewebearten.

## Patentansprüche

1. Vorrichtung zur Erkennung von tumorösem Gewebe mit mindestens einer ersten Anregungslichtquelle (12), wobei die erste Anregungslichtquelle (12) ein erstes Anregungslicht (34) einer Wellenlänge zwischen 300 - 314 nm aussendet und mindestens einem Lichtleiter (14) zur Führung des ersten Anregungslichts (34) an ein Objektfeld (18) des zu untersuchenden Gewebes (16) aufweist und mit mindestens einem Objektiv (24) zur Abbildung eines mittels des ersten Anregungslichts (34) generierten Autofluoreszenzsignals und/oder Remissionssignals (20) des Gewebes (16) auf den CCD-Chip oder ICCD-Chip einer Kamera (22) sowie mindestens eine Datenverarbeitungsanlage (28) zur Verarbeitung der von der Kamera (22) übermittelten Signale, wobei das Objektiv (24) ein Dachkantprisma (26) sowie mindestens eine achromatische UV-Linse (52, 54) aufweist und geeignet ist UV-Licht zu verarbeiten und derart ausgestaltet ist, dass mindestens zwei Bilder (48, 50) aus unterschiedlichen Spektralbereichen des fluoreszierenden Objektfelds (18) generiert und auf den CCD-Chip oder ICCD-Chip abgebildet werden, wobei mindestens jeweils ein Bild (48, 50) den UV-Bereich und einen anderen, davon verschiedenen Wellenlängen-Bereich des Autofluoreszenzsignals und/oder des Remissionssignals (20) des Objektfelds (18) darstellen.

2. Vorrichtung nach Anspruch 1,
**dadurch gekennzeichnet,**
**daß** die Vorrichtung (10) eine zweite Anregungslichtquelle zur Aussendung von einem zweiten Anregungslicht mit einer Wellenlänge zwischen 312 - 340 nm und/oder 350-410 nm aufweist.

3. Vorrichtung nach Anspruch 1 oder 2,
**dadurch gekennzeichnet,**
**daß** die erste Anregungslichtquelle (12) ein XeF-, XeCl- oder HeCd-Laser ist.

4. Vorrichtung nach Anspruch 2 oder 3,
**dadurch gekennzeichnet,**
**daß** die zweite Anregungslichtquelle eine Xe-, Hg- oder Deuterium-Lampe oder ein N₂-Laser ist.

5. Verfahren zur Erkennung von tumorösem Gewebe, wobei das Verfahren folgende Schritte umfasst:
a) Bestrahlung von Gewebe (16) mit einem ersten Anregungslicht (34) einer Wellenlänge zwischen 300 - 314 nm einer ersten Anregungslichtquelle (12) und Generierung einer Autofluoreszenz und/oder Remission eines Objektfelds (18) des bestrahlten Gewebes (16);
b) Generierung von mindestens zwei Bildern (48, 50) aus unterschiedlichen Spektralbereichen des fluoreszierenden Objektfelds (18) mittels eines Objektivs (24) einer Kamera (22), wobei das Objektiv (24) ein Dachkantprisma (26) sowie mindestens eine achromatische UV-Linse (52, 54) aufweist; und Abbildung der mindestens zwei Bilder (48, 50) auf den CCD-Chip oder ICCD-Chip der Kamera (22), wobei mindestens jeweils ein Bild (48, 50) den UV-Bereich und einen anderen, davon verschiedenen Wellenlängen-Bereich des Autofluoreszenzsignals (20) und/oder des Remissionssignals des Objektfelds (18) darstellen;
c) Übermittlung der in der Kamera (22) generierten Bild-/Videosignale an eine Datenverarbeitungsanlage (28);
d) Subtraktion von Backgroundsignalen von den generierten Bild-/Videosignalen;
e) Einlesen und Verrechnen des UV-Bildes (48) in und mit einem Blaukanal und Einlesen und Verrechnen des anderen Bildes (50) in und mit einem Grün- und/oder Rotkanal;
f) Verstärkung oder Abschwächung der einzelnen Farbkanäle zur Erzielung einer Standard-Farbeinstellung für normales, nicht-tumoröses Gewebe; und
g) Auswertung der farbcodierten Bilder (48, 50) bzw. deren farbcodierten Bild-/Videosignalen (32) zur Unterscheidung von normalem, gesundem Gewebe von tumorösem Gewebe.

6. Verfahren nach Anspruch 5,
**dadurch gekennzeichnet,**
**daß** im Verfahrensschritt b) zwei Bilder generiert werden, wobei ein Bild (48) den UV-Bereich und das andere Bild (50) den sichtbaren Wellenlängen-Bereich des Autofluoreszenzsignals und/oder des Remissionssignals (20) des Objektfelds (18) darstellt.

7. Verfahren nach Anspruch 5,
**dadurch gekennzeichnet,**
**daß** im Verfahrensschritt a) das Gewebe (16) mit der ersten Anregungslichtquelle (34) und einer zweiten Anregungslichtquelle zur Aussendung von einem zweiten Anregungslicht mit einer Wellenlänge zwischen 312 - 340 nm und/oder 350 - 410 nm bestrahlt wird.

8. Verfahren nach Anspruch 7,
**dadurch gekennzeichnet,**
**daß** im Verfahrensschritt b) zwei Bilder generiert werden, wobei ein erstes Bild (48) den UV-Bereich und ein anderes Bild den Infrarot-Bereich des Autofluoreszenzsignals und/oder des Remissionssignals (20) des Objektfelds (18) darstellt.

9. Verfahren nach Anspruch 7,
**dadurch gekennzeichnet,**
**daß** im Verfahrensschritt b) drei Bilder generiert werden, wobei ein erstes Bild (48) den UV-Bereich, das zweite Bild (50) den sichtbaren Wellenlängen-Bereich und das dritte Bild den Infrarot-Bereich des Autofluoreszenzsignals und/oder des Remissionssignals (20) des Objektfelds (18) darstellt.

10. Verfahren nach einem der Ansprüche 5 bis 9,
**dadurch gekennzeichnet,**
**daß** die Auswertung der farbcodierten Bilder bzw. farbcodierten Bild-/Videosignale im Verfahrensschritt g) durch Vergleich und Darstellung der generierten Standard-Farbeinstellung für normales, nicht-tumoröses Gewebe mit den generierten Farbeinstellungen für tumoröses Gewebe erfolgt.

11. Verfahren nach einem der Ansprüche 5 bis 9,
**dadurch gekennzeichnet,**
**daß** die Auswertung der farbcodierten Bilder bzw. farbcodierten Bild-/Videosignale im Verfahrensschritt g) folgende Schritte umfasst:
g1) Aufheben der Gammakorrektur durch eine Korrekturfunktion und Linearisierung der Farbkanäle;
g2) Verhältnisbildung der Intensitäten der Farbkanäle und Logarithmierung des Ergebnisses, wobei eine Charakterisierung des Gewebes dahingehend erfolgt, daß positive Werte tumoröses Gewebe und negative Werte normales, gesundes Gewebe darstellen; und
g3) Mittelung der Helligkeitswerte der Farbkanäle und Verrechnung mit einem in Verfahrensschritt g2) erstellten Ratiobild zur Darstellung der Geometrie der Verteilung der Gewebearten.

## Claims

1. Device for detecting tumorous tissue including at least a first excitation light source (12), the first excitation light source (12) emitting first excitation light (34) of a wavelength between 300 and 314 nm, and at least one light guide (14) for guiding the first excitation light (34) to an object field (18) of the tissue (16) to be examined, and including at least one objective (24) for imaging an autofluorescence signal and/or remission signal (20) of the tissue (16) generated by means of the first excitation light (34) onto the CCD chip or ICCD chip of a camera (22), as well as at least one data processing system (28) for processing the signals transmitted by the camera (22), wherein the objective (24) has a pentaprism (26) as well as at least one achromatic UV lens (52, 54), and is suitable to process UV light and designed such that at least two images (48, 50) are generated from different spectral ranges of the fluorescing object field (18) and imaged onto the CCD chip or ICCD chip, wherein at least each one image (48, 50) represents the UV range and another wavelength range, different from that, of the autofluorescence signal and/or the remission signal (20) of the object field (18).

2. Device according to claim 1,
**characterized in that**
the device (10) has a second excitation light source for emitting second excitation light with a wavelength between 312 and 340 nm and/or 350 and 410 nm.

3. Device according to claim 1 or 2,
**characterized in that**
the first excitation light source (12) is a XeF, XeCl or HeCd laser.

4. Device according to claim 2 or 3,
**characterized in that**
the second excitation light source is a Xe, Hg or deuterium lamp or an N₂ laser.

5. Method for detecting tumorous tissue, the method including the following steps:
a) irradiating tissue (16) with first excitation light (34) of a wavelength between 300 and 314 nm of a first excitation light source (12) and generating autofluorescence and/or remission of an object field (18) of the irradiated tissue (16);
b) generating at least two images (48, 50) from different spectral ranges of the fluorescing object field (18) by means of an objective (24) of a camera (22), wherein the objective (24) has a pentaprism (26) as well as at least one achromatic UV lens (52, 54); and imaging the at least two images (48, 50) onto the CCD chip or ICCD chip of the camera (22), wherein at least each one image (48, 50) represents the UV range and another wavelength range, different from that, of the auto-fluorescence signal (20) and/or the remission signal of the object field (18);
c) transmitting the image/video signals generated in the camera (22) to a data processing system (28);
d) subtracting background signals from the generated image/video signals;
e) reading-in and calculating the UV image (48) in and with a blue channel and reading-in and calculating the other image (50) in and with a green and/or red channel;
f) amplifying or attenuating the individual color channels for achieving standard color setting for normal, non-tumorous tissue; and
g) evaluating the color-coded images (48, 50) or the color-coded image/video signals (32) thereof, respectively, for differentiating normal, healthy tissue from tumorous tissue.

6. Method according to claim 5,
**characterized in that**
two images are generated in method step b), wherein one image (48) represents the UV range and the other image (50) represents the visible wavelength range of the autofluorescence signal and/or the remission signal (20) of the object field (18).

7. Method according to claim 5,
**characterized in that**
in method step a), the tissue (16) is irradiated with the first excitation light source (34) and a second excitation light source for emitting second excitation light with a wavelength between 312 and 340 nm and/or 350 and 410 nm.

8. Method according to claim 7,
**characterized in that**
two images are generated in method step b), wherein a first image (48) represents the UV range and another image represents the infrared range of the autofluorescence signal and/or the remission signal (20) of the object field (18).

9. Method according to claim 7,
**characterized in that**
three images are generated in method step b), wherein a first image (48) represents the UV range, the second image (50) represents the visible wavelength range and the third image represents the infrared range of the autofluorescence signal and/or the remission signal (20) of the object field (18).

10. Method according to any one of claims 5 to 9,
**characterized in that**
the evaluation of the color-coded images or color-coded image/video signals in method step g), respectively, is effected by comparison and representation of the generated standard color setting for normal, non-tumorous tissue with the generated color settings for tumorous tissue.

11. Method according to any one of claims 5 to 9,
**characterized in that**
the evaluation of the color-coded images or color-coded image/video signals in method step g), respectively, includes the following steps:
g1) canceling the gamma correction by a correction function and linearization of the color channels;
g2) formation of the ratio of the intensities of the color channels and logarithmation of the result, wherein characterization of the tissue is effected in that positive values represent tumorous tissue and negative values represent normal, healthy tissue; and
g3) averaging the brightness values of the color channels and calculating with a ratio image created in method step g2) for representing the geometry of the distribution of the tissue types.

## Revendications

1. Dispositif de reconnaissance de tissu tumeureux comprenant au moins une première source lumineuse d'excitation (12), la première source lumineuse d'excitation (12) émettant une première lumière d'excitation (34) d'une longueur d'onde comprise entre 300 et 314 nm et au moins une fibre optique (14) pour le guidage de la première lumière d'excitation (34) vers un champ d'objet (18) du tissu à analyser (16), et au moins un objectif (24) pour représenter un signal d'autofluorescence et/ou un signal de rémission (20) du tissu (16) généré à l'aide de la première lumière d'excitation (34) sur une puce CCD ou ICCD d'une caméra (22) ainsi qu'au moins une installation de traitement des données (28) pour le traitement des signaux transmis par la caméra (22), l'objectif (24) comprenant un prisme triangulaire (26) ainsi qu'au moins une lentille UV achromatique (52, 54) et étant apte à traiter de la lumière UV et conçu de manière à ce qu'au moins deux images (48, 50) de gammes spectrales differentes du champ d'objet (18) fluorescent soient générées et représentées sur la puce CCD ou ICCD, au moins une image (48, 50) chacune représentant la gamme UV et une autre gamme de longueur d'onde différente de ce premier du signal d'autofluorescence et/ou de rémission (20) du champ d'objet (18).

2. Système selon la revendication 1, **caractérisé en ce que** le dispositif (10) comporte une seconde source lumineuse d'excitation pour émettre une seconde lumière d'excitation à une longueur d'onde comprise entre 312 et 340 nm et/ou comprise entre 350 et 410 nm.

3. Système selon la revendication 1 ou 2, **caractérisé en ce que** la première source lumineuse d'excitation (12) est un laser XeF, XeCl ou HeCd.

4. Système selon la revendication 2 ou 3, **caractérisé en ce que** la seconde source lumineuse d'excitation est soit une lampe Xe, Hg ou deutérium soit un laser N₂.

5. Procéde de reconnaissance de tissu tumeureux, le procédé comprenant les étapes suivantes:
a) exposition de tissu (16) aux rayons au moyen d'une première lumière d'excitation (34) d'une longueur d'onde comprise entre 300 et 314 nm d'une première source lumineuse d'excitation (12) et génération d'une autofluorescence et/ou d'une rémission d'un champ d'objet (18) du tissu (16) exposé aux rayons;
b) génération d'au moins deux images (48, 50) sur des gammes spectrales différentes du champ d'objet fluorescent (18) moyennant un objectif (24) d'une caméra (22), l'objectif (24) comportant un prisme triangulaire (26) ainsi qu'au moins une lentille UV achromatique (52, 54); et représentation des au moins deux images (48, 50) sur la puce CCD ou ICCD de la caméra (22), au moins une image (48, 50) chacune représentant la gamme UV et une gamme de longueur d'onde différente de celle du signal d'autofluorescence (20) et/ou du signal de rémission du champ d'objet (18);
c) transmission des signaux d'image/vidéo générés dans la caméra (22) à une installation de traitement des données (28);
d) soustraction de signaux de fond des signaux d'image/vidéo générés;
e) lecture de l'image UV (48) dans un canal bleu et calcul de celle-ci par rapport à ce dernier et lecture de l'autre image (50) dans un canal vert et/ou rouge et calcul de celle-ci par rapport à ce dernier;
f) renforcement ou atténuation des différents canaux de couleur pour obtenir un réglage de couleurs standard pour du tissu normal non tumeureux; et
g) dépouillement des images codées en couleur (48, 50) resp. de leurs signaux d'image/vidéo codés en couleur (32) afin de distinguer le tissu normal d'une personne en bonne santé de tissus tumeureux.

6. Procédé selon la revendication 5, **caractérisé en ce que** dans l'étape du procédé b), il y a génération de deux images, une image (48) représentant la gamme UV, l'autre image (50) représentant la gamme de longueur d'onde visible du signal d'autofluorescence et/ou de rémission (20) du champ d'objet (18).

7. Procédé selon la revendication 5, **caractérisé en ce que** dans l'étape du procédé a), le tissu est exposé aux rayons de la première source lumineuse d'excitation (34) et d'une seconde source lumineuse d'excitation pour l'émission d'une seconde lumière d'excitation ayant une longueur d'onde comprise entre 312 et 340 nm et/ou entre 350 et 410 nm.

8. Procédé selon la revendication 7, **caractérisé en ce que** que dans l'étape du procédé b), il y a génération de deux images, une première image (48) représentant la gamme UV et une autre image représentant la gamme infrarouge du signal d'autofluorescence et/ou de rémission (20) du champ d'objet (18).

9. Procédé selon la revendication 7, **caractérisé en ce que** que dans l'étape du procédé b), il y a génération de trois images, une première image (48) représentant la gamme UV, la seconde image (50) représentant la gamme de longueur d'onde visible et la troisième image représentant la gamme infrarouge du signal d'autofluorescence et/ou de rémission (20) du champ d'objet (18).

10. Procédé selon l'une des revendications 5 à 9, **caractérisé en ce que** le dépouillement des images codées en couleur respectivement des signaux d'image/vidéo codés en couleur se fait dans l'étape du procédé g) par comparaison et représentation du réglage en couleur standard généré pour du tissu normal non pas tumeureux avec les réglages en couleur générés pour du tissu tumeureux.

11. Procédé selon l'une des revendications 5 à 9, **caractérisé en ce que** le dépouillement des images codées en couleur respectivement des signaux d'image/vidéo codés en couleur comprend, dans l'étape du procédé g), les étapes suivantes:
g1) suppression de la correction gamma à l'aide d'une fonction de correction et linéarisation des canaux de couleur;
g2) formation de rapports des intensités des canaux de couleurs et logarithmisation du résultat, une caractérisation du tissu étant faite en ce sens que les valeurs positives représentent du tissu tumeureux et les valeurs négatives représentent du tissu normal d'une personne non pas malade; et
g3) moyennisation des valeurs de luminosité des canaux de couleurs et calcul par rapport à une image "ratio" établie dans l'étape du procédé g2) pour représenter la géometrie de la répartition des espèces de tissu.
